# EUROPEAN PATENT APPLICATION

(11) **EP 1 881 672 A1**
(43) Date of publication of application: **23.01.2008**
(21) Application number: 07107265.6
(22) Date of filing: 30.04.2007
(51) Int. Cl.: H04L 29/06, G06F 17/30, G06F 19/00, G06F 21/00

(54) **Ultrasonic moving-picture real-time service system and method and recording medium having embodied thereon computer program for performing method**

(30) Priority: 03.05.2006 KR 20060040099; 28.12.2006 KR 20060135833
(71) Applicant: Medinbiz Co., Ltd., Seongsu-dong 2ga, Seongdong-gu 133-120 Seoul (KR)
(72) Inventor: KIM, Nam Ju, 133-110, Seoul (KR)
(74) Representative: Walaski, Jan Filip

(57) **Abstract**

Disclosed herein are an ultrasonic moving-picture real-time service system and method and a recording medium having embodied thereon a computer program for performing the method and, more particularly, to an ultrasonic moving-picture real-time service system and method and a recording medium having embodied thereon a computer program for performing the method, which can allow the family of a pregnant woman to view an ultrasonic moving picture in real time over an Internet network while the pregnant woman receives a medical treatment using ultrasonic waves in a hospital and can automatically log in to a central server of the hospital and stream or download an ultrasonic moving picture of the pregnant woman when a compact disc (CD), which receives from the hospital and stores basic contents and a serial number, is inserted into a personal computer (PC) which can access to the Internet.

The ultrasonic moving-picture real-time service method includes (a) storing basic contents and a serial number for using an ultrasonic moving-picture real-time service over an Internet network in a compact disc (CD) and attaching a barcode corresponding to the serial number to a surface of the CD; (b) at a personal computer (PC), logging in to an ultrasonic moving-picture real-time service program and inputting the barcode attached to the surface of the CD to a barcode reader connected to the PC; (c) pressing a start button of an ultrasonic picture service in the ultrasonic moving-picture real-time service program, photographing an ultrasonic picture, and transmitting the photographed ultrasonic picture and the barcode to a central server connected to the PC; (d) at the central server, storing the received ultrasonic moving picture in correspondence with the serial number corresponding to the barcode; (e) at a PC which can access to the central server over an Internet communication network, reading the basic contents stored on the CD by insertion of the CD and automatically accessing to the central server; and (f) streaming or downloading the moving picture corresponding to the serial number stored on the CD from the central server.

## Description

### BACKGROUND OF THE INVENTION

### Field of the Invention

The present invention relates to an ultrasonic moving-picture real-time service system and method and a recording medium having embodied thereon a computer program for performing the method, and more particularly, to an ultrasonic moving-picture real-time service system and method and a recording medium having embodied thereon a computer program for performing the method, which can allow the family of a pregnant woman to view an ultrasonic moving picture in real time over an Internet network while the pregnant woman receives a medical treatment using ultrasonic waves in a hospital and can automatically log in to a central server of the hospital and stream or download an ultrasonic moving picture of the pregnant woman when a compact disc (CD), which receives from the hospital and stores basic contents and a serial number, is inserted into a personal computer (PC) which can access to the Internet.

### Description of the Related Art

Recently, when a pregnant woman receives a medical treatment using ultrasonic waves, an obstetrics and gynecology hospital provides a compact disc (CD) for storing an ultrasonic moving picture as a service for a customer. When the pregnant woman brings the CD to the hospital upon her next medical treatment, the ultrasonic moving pictures which are photographed on a treatment date are stored on the CD.

In a case where the photographed ultrasonic pictures are stored on the CD, since basic contents for viewing the ultrasonic moving pictures are stored on the CD, it is advantageous that the family of the pregnant woman can view the moving pictures using their computer without streaming contents for viewing the moving pictures from a server. In addition, it is advantageous that the pregnant woman directly receives an identifiable product such as a CD title.

However, in a method of storing the ultrasonic moving pictures of the pregnant woman on the CD at the hospital, problems such as data error and recognition rate deterioration are caused due to a time for recognizing the CD and time for writing data when recording customer contents and repeated writing of data on one CD whenever the medical treatment is performed.

In a case of using an Internet service instead of the CD, the CD title cannot be provided to the pregnant woman and the basic contents which are commonly used by all the customers must be streamed or downloaded. In addition, when the customer inputs a serial number (for example, key values of a customer such as a resident registration number, a telephone number and so on), which is used to allow the customer (pregnant woman) to access to an ultrasonic moving picture of the customer, using a medical-office PC or when the customer inputs the serial number using the Internet, the serial number may be input erroneously. Moreover, it is troublesome that a serial number is individually input.

Conventionally, unless the family of the pregnant woman goes to the hospital together with the pregnant woman, the family of the pregnant woman cannot view the ultrasonic moving pictures which are photographed in the hospital in real time.

In addition, unless the pregnant woman receives the CD for storing the ultrasonic moving pictures from the hospital and transmits them to her husband or family staying at a remote place using a PC, the moving pictures cannot be viewed.

### SUMMARY OF THE INVENTION

Therefore, the present invention has been made in view of the above problems. It is an object of the present invention to provide an ultrasonic moving-picture real-time service system and method and a recording medium having embodied thereon a computer program for performing the method, which can allow the family of a pregnant woman to view an ultrasonic moving picture in real time over an Internet network while the pregnant woman receives a medical treatment using ultrasonic waves in a hospital.

It is another object of the present invention to provide an ultrasonic moving-picture real-time service system and method and a recording medium having embodied thereon a computer program for performing the method, which can automatically log in to a central server of a hospital and stream or download an ultrasonic moving picture of a pregnant woman when a compact disc (CD), which receives from the hospital and stores basic contents and a serial number, is inserted into a personal computer (PC) which can access to the Internet.

It is another object of the present invention to provide an ultrasonic moving-picture real-time service system and method and a recording medium having embodied thereon a computer program for performing the method, which can stream or download an ultrasonic moving picture over an Internet network by reading a barcode attached to a CD using a barcode reader connected to a PC, photographing the ultrasonic picture, transmitting the photographed ultrasonic picture to a central server of a hospital, and storing the ultrasonic moving picture together with a serial number corresponding to the read barcode, when a pregnant woman brings the CD for storing the basic contents and the serial number to the hospital and a doctor performs a medical treatment using ultrasonic waves.

In accordance with an aspect of the present invention, the above and other objects can be accomplished by the provision of an ultrasonic moving-picture real-time service method comprising: (a) storing basic contents and a serial number for using an ultrasonic moving-picture real-time service over an Internet network in a compact disc (CD) and attaching a barcode corresponding to the serial number to a surface of the CD; (b) at a personal computer (PC), logging in to an ultrasonic moving-picture real-time service program and inputting the barcode attached to the surface of the CD to a barcode reader connected to the PC; (c) pressing a start button of an ultrasonic picture service in the ultrasonic moving-picture real-time service program, photographing an ultrasonic picture, and transmitting the photographed ultrasonic picture and the barcode to a central server connected to the PC; (d) at the central server, storing the received ultrasonic moving picture in correspondence with the serial number corresponding to the barcode; (e) at a PC which can access to the central server over an Internet communication network, reading the basic contents stored on the CD by insertion of the CD and automatically accessing to the central server; and (f) streaming or downloading the moving picture corresponding to the serial number stored on the CD from the central server.

The method may further comprise (g) at the central server, performing member admission and registration through the ultrasonic moving-picture real-time service contents over the Internet communication network; (h) receiving an identity (ID) and a password and performing an authentication process when accessing to the ultrasonic moving-picture real-time service contents; (i) receiving the serial number stored on the CD and performing an authentication process; and (j) streaming or downloading the ultrasonic moving picture corresponding to the serial number.

The method may further comprise (b1) generating a signature input window for inputting an electronic signature on a monitor of the PC when the barcode of the CD is input through the barcode reader in the step (b); and (b2) allowing the moving picture to be downloaded when a signature is input to the signature input window.

In accordance with another aspect of the present invention, there is provided an ultrasonic moving-picture real-time service system comprising: a compact disc (CD) in which basic contents and a serial number for using an ultrasonic moving-picture real-time service over the Internet are stored and a barcode corresponding to the serial number is attached to a surface thereof; a plurality of personal computers (PCs) which is connected to a medical apparatus including an ultrasonic photographing apparatus for photographing an ultrasonic picture and a barcode reader for inputting the barcode of the CD, logs in to an ultrasonic moving-picture real-time service program stored therein, and presses a start button of an ultrasonic picture service to transmit the photographed ultrasonic picture and the barcode to a central server; the central server which receives the ultrasonic picture and the barcode from the plurality of PCs over a communication network, stores the received ultrasonic picture in correspondence with the serial number corresponding to the barcode, performs a member authentication process using a user PC which accesses to the central server over an Internet network, and allows the ultrasonic moving picture corresponding to the serial number to be streamed or downloaded to the user PC; and a plurality of customer PCs which reads the basic contents stored on the CD by insertion of the CD, automatically accesses to the ultrasonic moving-picture real-time service contents, and streams or downloads the ultrasonic moving picture corresponding to the serial number stored on the CD.

The plurality of customer PCs may access the ultrasonic moving-picture real-time service contents over the Internet network, receive data necessary for member admission from a user according to a guide of the ultrasonic moving-picture real-time service contents, and transmit the data to the central server; receive an identity (ID) and a password, transmits the ID and the password to the central server, and perform an authentication process, after the member admission is completed; and receive the serial number, transmit the serial number to the central server, and stream or download the ultrasonic moving picture corresponding to the serial number, after an authentication process is performed at the central server.

The central server may comprise a moving picture service application which stores the ultrasonic moving-picture real-time service program; a serial number generation unit which generates the serial number; a barcode generation unit which generates the barcode corresponding to the serial number; a basic content storage unit which stores the basic contents which will be stored on the CD; a member authentication unit which compares an identity (ID) and a password, which are input by a member, with an ID and a password, which are previously stored, and performs a member authentication process; an Internet access module which functions as an interface for accessing to the Internet network; and a central processing unit (CPU) which controls the operations of components of the central server.

The central sever may comprise a member information database (DB) which stores member information; and a moving picture information DB which stores the ultrasonic moving picture in correspondence with the serial number.

In accordance with another aspect of the present invention, there is provided a recording medium having embodied thereon a computer program for performing the ultrasonic moving-picture real-time service method according to any one of claims 1 to 3.

### BRIEF DESCRIPTION OF THE DRAWINGS

The above and other objects, features and other advantages of the present invention will be more clearly understood from the following detailed description taken in conjunction with the accompanying drawings, in which:

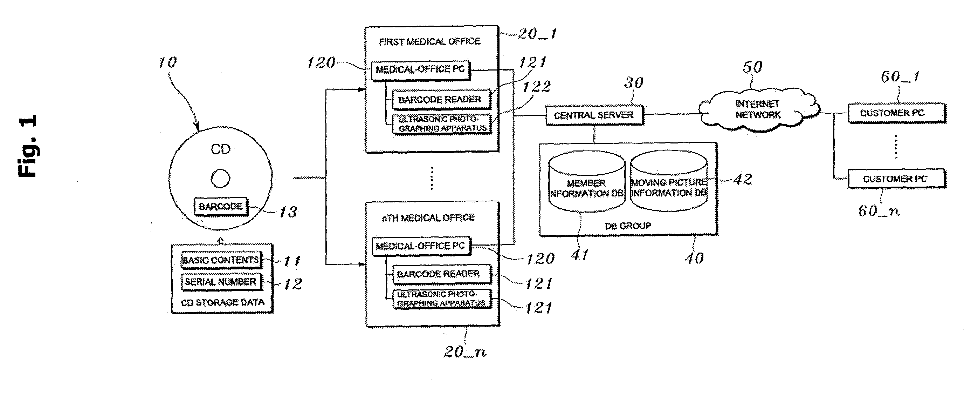

FIG. 1 is a block diagram showing the configuration of an ultrasonic moving-picture real-time service system according to a preferred embodiment of the present invention;

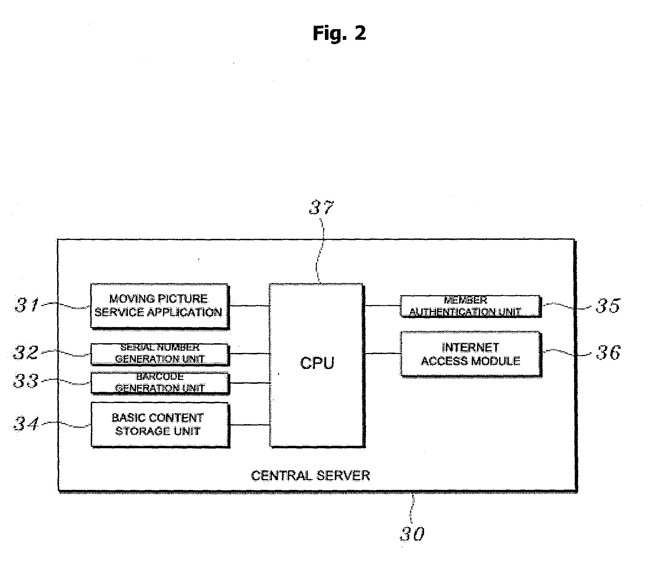

FIG. 2 is a block diagram showing the configuration of a central server shown in FIG. 1;

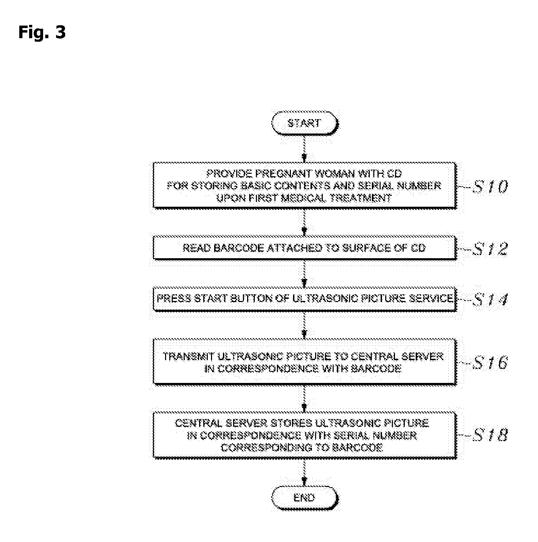

FIG. 3 is a flowchart illustrating a method of reading a barcode attached to a CD, which is provided from a hospital for providing an ultrasonic moving-picture real-time service to a pregnant woman upon a medical treatment, and photographing and storing an ultrasonic moving picture, according to the present invention;

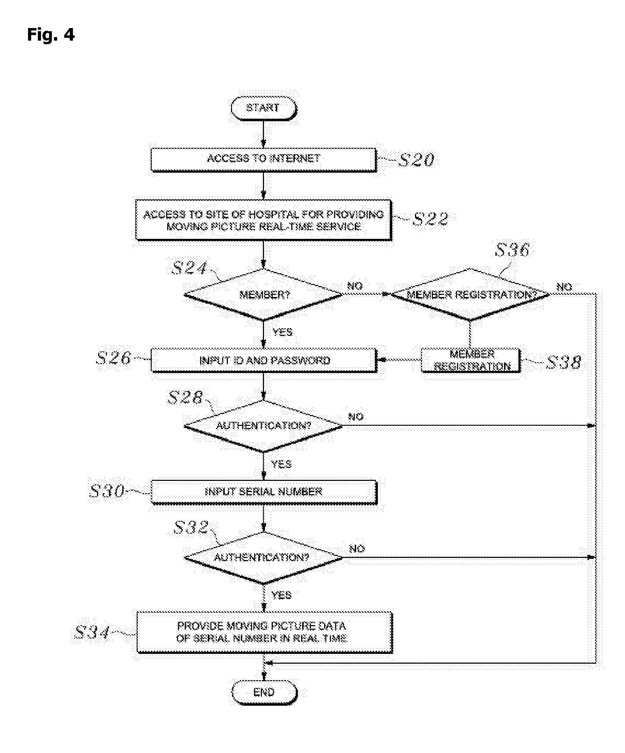

FIG. 4 is a flowchart illustrating a method of allowing the family of the pregnant woman to access to an Internet site of the hospital for providing the ultrasonic moving-picture real-time service and to view the ultrasonic moving picture of the pregnant woman in real time, according to the present invention; and

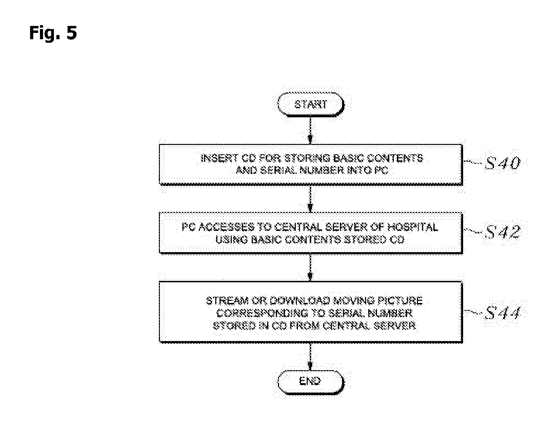

FIG. 5 is a flowchart illustrating a method of inserting the CD, which is provided by the hospital for providing the ultrasonic moving-picture real-time service, into a PC and streaming or downloading the ultrasonic moving picture of the pregnant woman stored in the central server of the hospital, according to the present invention.

### DESCRIPTION OF THE PREFERRED EMBODIMENTS

### ULTRASONIC MOVING-PICTURE REAL-TIME SERVICE SYSTEM

FIG. 1 is a block diagram showing the configuration of an ultrasonic moving-picture real-time service system according to a preferred embodiment of the present invention.

As shown in FIG. 1, the ultrasonic moving-picture real-time service system according to the present invention includes a CD 10 in which basic contents 11 and a serial number 12 are stored and a barcode 13 is attached to a surface thereof, first to n^{th} medical offices 20_1 to 20_n respectively including a plurality of medical-office PCs 120 connected to a barcode reader 121 and an ultrasonic photographing apparatus 122 and so on, a central server 30, a database (DB) group 40 including a member information DB 41 and a moving picture information DB 42, an Internet network 50, and a plurality of customer PCs 60-1 to 60-n.

The CD 10 stores the basic contents 11 and the serial number 12 which can use an ultrasonic moving-picture real-time service over the Internet. The barcode 13 corresponding to the serial number 12 is attached to the surface of the CD. A hospital provides the CD 10 to a pregnant woman who first receives a medical treatment using ultrasonic waves.

Each of the plurality of medical-office PCs 120 logs in to an ultrasonic moving-picture real-time service program stored therein, inputs the barcode 13 of the CD 10 to the barcode reader 121, and transmits a photographed ultrasonic moving picture and the barcode to the central server 30 of the hospital over a communication network when pressing a start button of an ultrasonic moving picture service.

The central server 30 receives the ultrasonic moving picture and the barcode from each of the plurality of medical-office PCs 120 over the communication network, stores the received ultrasonic moving picture and the serial number corresponding to the barcode, manages ultrasonic moving-picture real-time service contents over the Internet network, performs a member authentication process of the user who accesses to the central server over the Internet network, and allows the user to stream or download the ultrasonic moving picture corresponding to the input serial number. The central server 30 includes a member information DB 41 for storing member information and a moving picture information DB 42 for storing the ultrasonic moving picture in correspondence with the serial number.

When the CD 10 is inserted into each of the plurality of customer PCs 60_1 to 60_n, each of the plurality of customer PCs 60_1 to 60_n automatically accesses to the ultrasonic moving-picture real-time service contents of the hospital using the basic contents stored on the CD 10 and streams or downloads the ultrasonic moving picture corresponding to the serial number stored on the CD 10.

### CENTRAL SERVER 30

FIG. 2 is a block diagram showing the configuration of the central server 30 shown in FIG. 1.

As shown in fig. 2, the central server 30 includes a moving-picture service application 31, a serial number generation unit 32, a barcode generation unit 33, a basic content storage unit 34, a member authentication unit 35, an Internet access module 36 and a CPU 37.

The moving picture service application 32 stores the ultrasonic moving-picture real-time service program and the serial number generation unit 32 generates the serial number corresponding to the barcode. The barcode generation unit 33 generates the barcode corresponding to the serial number and the basic content storage unit 34 stores the basic contents which will be stored on the CD 10. The member authentication unit 35 compares an identity (ID) and a password, which are input by a member, with an ID and a password, which are previously stored, and performs a member authentication process. The Internet access module 36 functions as an interface for accessing to the Internet network and the CPU 37 controls the operations of the components of the central server 30.

In the ultrasonic moving-picture real-time service system according to the preferred embodiment of the present invention having the above-described configuration, the barcode reader 121 and the ultrasonic photographing apparatus 122 are connected to each of the medical-office PCs 120 which are connected to the central server 30 of the hospital over the communication network and perform Internet communication. The CD 10 in which basic contents and the serial number are previously stored and the barcode corresponding to the serial number is attached to the surface thereof is provided to the customer (pregnant woman) who goes to the hospital.

When the pregnant woman goes to the hospital with the CD 10 which is received from the hospital, a doctor inputs the barcode attached to the CD 10 to the barcode reader 121 when the medical treatment using the ultrasonic waves starts. At this time, the medical-office PC 120 logs in to the ultrasonic moving-picture real-time service program.

Subsequently, when the start button of the ultrasonic moving-picture service provided by the ultrasonic moving-picture real-time service program is pressed, the ultrasonic moving picture is photographed, and the photographed ultrasonic picture and the barcode are transmitted to the central server 30 of the hospital.

The central server 30 stores the ultrasonic moving picture received from the medical-office PC 120 in the moving picture DB 42 in correspondence with the serial number corresponding to the barcode.

Thereafter, when the pregnant woman (customer) who returns home after the medical treatment inserts the CD 10 into the PC which can access to the Internet, the pregnant woman automatically accesses the ultrasonic moving-picture real-time service contents using the basic contents stored on the CD and streams or downloads the ultrasonic moving picture corresponding to the serial number stored on the CD 10 from the central server 30.

### METHOD OF STORING ULTRASONIC MOVING PICTURE UPON MEDICAL TREATMENT

FIG. 3 is a flowchart illustrating a method of reading the barcode attached to the CD, which is provided from the hospital for providing the ultrasonic moving-picture real-time service to the pregnant woman upon the medical treatment, and photographing and storing the ultrasonic moving picture, according to the present invention.

As shown in Fig. 3, in the method of storing the ultrasonic moving picture, the CD 10 in which the basic contents and the serial number, which can use the ultrasonic moving-picture real-time service through the Internet network, are stored and the barcode corresponding to the serial number is attached to the surface thereof is manufactured and provided to the pregnant woman who receives the medical treatment in the hospital (step S10).

Next, the medical-office PC 120 of the hospital logs in to the ultrasonic moving-picture real-time service program and reads the barcode 13 of the CD 10 of the pregnant woman using the barcode reader 121 connected to the medical-office PC 120 (step S12).

Next, the start button of the ultrasonic picture service is pressed in the ultrasonic moving-picture real-time service program (step S14), the ultrasonic moving picture is photographed, and the photographed ultrasonic picture and the barcode are transmitted to the central server 30 of the hospital (step S16).

Next, the central server 30 stores the ultrasonic moving picture in correspondence with the serial number corresponding to the barcode 13 (step S18).

Meanwhile, a step of opening a signature input window for inputting an electronic signature on a monitor of the medical-office PC 120 when the barcode 13 of the CD 10 is input to the barcode reader 121 in the step S12 and allowing the moving picture to be downloaded when the pregnant woman inputs the electronic signature to the signature input window may be further included.

### METHOD OF VIEWING ULTRASONIC MOVING PICTURE IN REAL TIME

FIG. 4 is a flowchart illustrating a method of allowing the family of the pregnant woman to access to an Internet site of the hospital for providing the ultrasonic moving-picture real-time service and to view the ultrasonic moving picture of the pregnant woman in real time, according to the present invention.

In the method of viewing the ultrasonic moving picture in real time, as shown in FIG. 4, the family of the pregnant woman accesses the ultrasonic moving-picture real-time service contents of the hospital over the Internet network (steps S20 to S22).

At this time, when the user is the member (in the step S24, yes), the ID and the password are input and the authentication process is performed (steps S24 to S28) and, when the user is not the member (in the step S24, no), member registration is performed (steps S36 and S38) and the ID and the password are input and the authentication process is performed (step S24 to S28).

Next, the serial number of the pregnant woman is input and the authentication process is performed (steps S30 and S32).

Next, the ultrasonic moving picture corresponding to the serial number is streamed or downloaded from the central server 30 (step S34).

### METHOD OF STREAMING OR DOWNLOADING ULTRASONIC MOVING PICTURE

FIG. 5 is a flowchart illustrating a method of inserting the CD, which is provided by the hospital for providing the ultrasonic moving-picture real-time service, into the PC and streaming or downloading the ultrasonic moving picture of the pregnant woman stored in the central server of the hospital, according to the present invention.

In the method of streaming or downloading the ultrasonic moving picture, as shown in FIG. 5, the pregnant woman who returns home after the medical treatment at the hospital inserts the CD into the PC which can access to the Internet (step S40) and automatically accesses the ultrasonic moving-picture real-time service contents of the hospital using the basic contents stored on the CD 10 (step S42).

Subsequently, the moving picture corresponding to the serial number stored on the CD 10 is streamed or downloaded from the central server 30 through the ultrasonic moving-picture real-time service contents (step S40).

As described above, according to an ultrasonic moving-picture real-time service system and method and a recording medium having embodied thereon a computer program of the present invention, it is possible to allow the family of a pregnant woman to view an ultrasonic moving picture in real time over an Internet network while the pregnant woman receives a medical treatment using ultrasonic waves in a hospital.

In addition, it is possible to automatically log in to a central server of the hospital and to stream or download the ultrasonic moving picture of the pregnant woman when a compact disc (CD), which receives from the hospital and stores basic contents and a serial number, is inserted into a personal computer (PC) which can access to the Internet.

Moreover, it is possible to stream or download the ultrasonic moving picture over an Internet network by reading a barcode attached to a CD using a barcode reader connected to the PC, photographing the ultrasonic picture, transmitting the photographed ultrasonic picture to a central server of hospital, and storing the ultrasonic moving picture together with a serial number corresponding to the read barcode, when a pregnant woman brings the CD for storing the basic contents and the serial number to the hospital and a doctor performs a medical treatment using ultrasonic waves.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.
FIG. 1
   13: BARCODE
   11: BASIC CONTENTS
   12: SERIAL NUMBER
   CD STORAGE DATA
   20_1: FIRST MEDICAL OFFICE
   120: MEDICAL-OFFICE PC
   121: BARCODE READER
   122: ULTRASONIC PHOTOGRAPHING APPARATUS
   20_n: n^{TH} MEDICAL OFFICE
   30: CENTRAL SERVER
   41: MEMBER INFORMATION DB
   42: MOVING PICTURE INFORMATION DB
   40: DB GROUP
   50: INTERNET NETWORK
   60_1, 60_n: CUSTOMER PC
FIG. 2
   31: MOVING PICTURE SERVICE APPLICATION
   32: SERIAL NUMBER GENERATION UNIT
   33: BARCODE GENERATION UNIT
   34: BASIC CONTENT STORAGE UNIT
   35: MEMBER AUTHENTICATION UNIT
   36: INTERNET ACCESS MODULE
   30: CENTRAL SERVER
FIG. 3
   START
   S10: PROVIDE PREGNANT WOMAN WITH CD FOR STORING BASIC CONTENTS AND SERIAL NUMBER UPON FIRST MEDICAL TREATMENT
   S12: READ BARCODE ATTACHED TO SURFACE OF CD
   S14: PRESS START BUTTON OF ULTRASONIC PICTURE SERVICE
   S16: TRANSMIT ULTRASONIC PICTURE TO CENTRAL SERVER IN CORRESPONDENCE WITH BARCODE
   S18: CENTRAL SERVER STORES ULTRASONIC PICTURE IN CORRESPONDENCE WITH SERIAL NUMBER CORRESPONDING TO BARCODE
   END
FIG. 4
   START
   S20: ACCESS TO SITE OF HOSPITAL FOR PROVIDING MOVING PICTURE REAL-TIME SERVICE
   S24: MEMBER?
   S26: INPUT ID AND PASSWORD
   S28: AUTHENTICATION?
   S30: INPUT SERIAL NUMBER
   S32: AUTHENTICATION?
   S34: PROVIDE MOVING PICTURE DATA OF SERIAL NUMBER IN REAL TIME
   S36: MEMBER REGISTRATION?
   S38: MEMBER REGISTRATION
   END
FIG. 5
   START
   S40: INSERT CD FOR STORING BASIC CONTENTS AND SERIAL NUMBER INTO PC
   S42: PC ACCESSES TO CENTRAL SERVER OF HOSPITAL USING BASIC CONTENTS STORED CD
   S44: STREAM OR DOWNLOAD MOVING PICTURE CORRESPONDING TO SERIAL NUMBER STORED IN CD FROM CENTRAL SERVER
   END

## Claims

1. An ultrasonic moving-picture real-time service method, comprising:
(a) storing basic contents and a serial number for using an ultrasonic moving-picture real-time service over an Internet network in a compact disc (CD) and attaching a barcode corresponding to the serial number to a surface of the CD;
(b) at a personal computer (PC), logging in to an ultrasonic moving-picture real-time service program and inputting the barcode attached to the surface of the CD to a barcode reader connected to the PC;
(c) pressing a start button of an ultrasonic picture service in the ultrasonic moving-picture real-time service program, photographing an ultrasonic picture, and transmitting the photographed ultrasonic picture and the barcode to a central server connected to the PC;
(d) at the central server, storing the received ultrasonic moving picture in correspondence with the serial number corresponding to the barcode;
(e) at a PC which can access to the central server over an Internet communication network, reading the basic contents stored on the CD by insertion of the CD and automatically accessing to the central server; and
(f) streaming or downloading the moving picture corresponding to the serial number stored on the CD from the central server.

2. The method according to claim 1, further comprising:
(g) at the central server, performing member admission and registration through the ultrasonic moving-picture real-time service contents over the Internet communication network;
(h) receiving an identity (ID) and a password and performing an authentication process when accessing to the ultrasonic moving-picture real-time service contents;
(i) receiving the serial number stored on the CD and performing an authentication process; and
(j) streaming or downloading the ultrasonic moving picture corresponding to the serial number.

3. The method according to claim 1, further comprising:
(b1) generating a signature input window for inputting an electronic signature on a monitor of the PC when the barcode of the CD is input through the barcode reader in the step (b); and
(b2) allowing the moving picture to be downloaded when a signature is input to the signature input window.

4. An ultrasonic moving-picture real-time service system, comprising:
a compact disc (CD) in which basic contents and a serial number for using an ultrasonic moving-picture real-time service over the Internet are stored and a barcode corresponding to the serial number is attached to a surface thereof;
a plurality of personal computers (PCs) which is connected to a medical apparatus including an ultrasonic photographing apparatus for photographing an ultrasonic picture and a barcode reader for inputting the barcode of the CD, logs in to an ultrasonic moving-picture real-time service program stored therein, and presses a start button of an ultrasonic picture service to transmit the photographed ultrasonic picture and the barcode to a central server;
the central server which receives the ultrasonic picture and the barcode from the plurality of PCs over a communication network, stores the received ultrasonic picture in correspondence with the serial number corresponding to the barcode, performs a member authentication process using a user PC which accesses to the central server over an Internet network, and allows the ultrasonic moving picture corresponding to the serial number to be streamed or downloaded to the user PC; and
a plurality of customer PCs which reads the basic contents stored on the CD by insertion of the CD, automatically accesses the ultrasonic moving-picture real-time service contents, and streams or downloads the ultrasonic moving picture corresponding to the serial number stored on the CD.

5. The system according to claim 4, wherein the plurality of customer PCs:
accesses the ultrasonic moving-picture real-time service contents over the Internet network, receives data necessary for member admission from a user according to a guide of the ultrasonic moving-picture real-time service contents, and transmits the data to the central server;
receives an identity (ID) and a password, transmits the ID and the password to the central server, and performs an authentication process, after the member admission is completed; and
receives the serial number, transmits the serial number to the central server, and streams or downloads the ultrasonic moving picture corresponding to the serial number, after an authentication process is performed at the central server.

6. The system according to claim 4, wherein the central server comprises:
a moving picture service application which stores the ultrasonic moving-picture real-time service program;
a serial number generation unit which generates the serial number;
a barcode generation unit which generates the barcode corresponding to the serial number;
a basic content storage unit which stores the basic contents which will be stored on the CD;
a member authentication unit which compares an identity (ID) and a password, which are input by a member, with an ID and a password, which are previously stored, and performs a member authentication process;
an Internet access module which functions as an interface for accessing to the Internet network; and
a central processing unit (CPU) which controls the operations of components of the central server.

7. The system according to claim 4 or 6, wherein the central sever comprises:
a member information database (DB) which stores member information; and
a moving picture information DB which stores the ultrasonic moving picture in correspondence with the serial number.

8. A recording medium having embodied thereon a computer program for performing the ultrasonic moving-picture real-time service method according to any one of claims 1 to 3.

9. A system comprising:
means for receiving a storage medium having a barcode, the barcode being associated with a serial number;
means for reading the barcode;
means for generating an image;
means for transmitting the image and the barcode to a server, the server comprising:
means for storing the image in correspondence with the serial number; and
means for sending the image to a user in response to receipt of a serial number corresponding to the image.

10. A system according to claim 9,
wherein the serial number is stored on the storage medium.
